(19) Europäisches Patentamt
European Patent Office
Office européen des brevets

(11) **EP 2 540 222 A2**

(12) **EUROPEAN PATENT APPLICATION**
published in accordance with Art. 153(4) EPC

(43) Date of publication:
**02.01.2013 Bulletin 2013/01**

(51) Int Cl.:
***A61B 5/0464*** (2006.01)　　　***A61B 5/04*** (2006.01)

(21) Application number: **11747749.7**

(22) Date of filing: **25.02.2011**

(86) International application number:
**PCT/KR2011/001360**

(87) International publication number:
**WO 2011/105859 (01.09.2011 Gazette 2011/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2010　KR 20100017665**

(71) Applicant: **Knu-Industry Cooperation Foundation Gangwon-do 200-090 (KR)**

(72) Inventors:
- **SHIM, Eun Bo**
  **Chuncheon-si**
  **Gangwon-do 200-170 (KR)**
- **LIM, Ki Moo**
  **Chuncheon-si**
  **Gangwon-do 200-160 (KR)**

(74) Representative: **Beier, Ralph**
  **v. Bezold & Partner**
  **Akademiestrasse 7**
  **80799 München (DE)**

(54) **METHOD AND APPARATUS FOR DIAGNOSING CARDIAC ARRHYTHMIA**

(57)　　　Disclosed is an arrhythmia diagnosing method and device for diagnosing arrhythmia such as fibrillation or tachycardia. The arrhythmia diagnosing method includes the steps of: (a) measuring a heart dimension; (b) measuring a frequency of a cardiac electric wave; (c) measuring a conduction velocity of the cardiac electric wave; and (d) determining occurrence or absence of arrhythmia using the heart dimension, the frequency of the cardiac electric wave and the conduction velocity of the cardiac electric wave measured in steps (a), (b) and (c). With the present invention, it is possible to predict and diagnose an electric wave tornado, one of causes of arrhythmia, through the use of a non-dimensional parameter, to find out patents who may possibly be died of arrhythmia or may possibly come to brain death due to arrhythmia and to significantly reduce the mortality rate of patients who suffer from arrhythmia.

Fig.7

EP 2 540 222 A2

## Description

Field of the Invention

The present invention relates to an arrhythmia diagnosing method and device and, more particularly, to an arrhythmia diagnosing method and device for diagnosing arrhythmia such as fibrillation or tachycardia.

Background of the Invention

A cardiac blood ejection activity is made by contraction and expansion of a heart. At this time, heart contraction is caused by an electric stimulation applied to myocardial cells and a resultant electric excitation. If the electric excitation occurs in the myocardial cells, the calcium concentration in the cells is changed. This activates contraction mechanisms existing in the cells, thereby inducing mechanical contraction. The heart has stimulus generating tissues (pacemakers) and stimulus transfer tissues (myocardial cells), both of which serve to generate the electric stimulation. In the stimulus generating tissues, electric stimulations are periodically generated at a frequency of from 60 to 100 per minute. If the electric stimulations are normally transferred to the myocardial cells, the heart repeats contraction and expansion, whereby the blood is supplied to individual body parts.

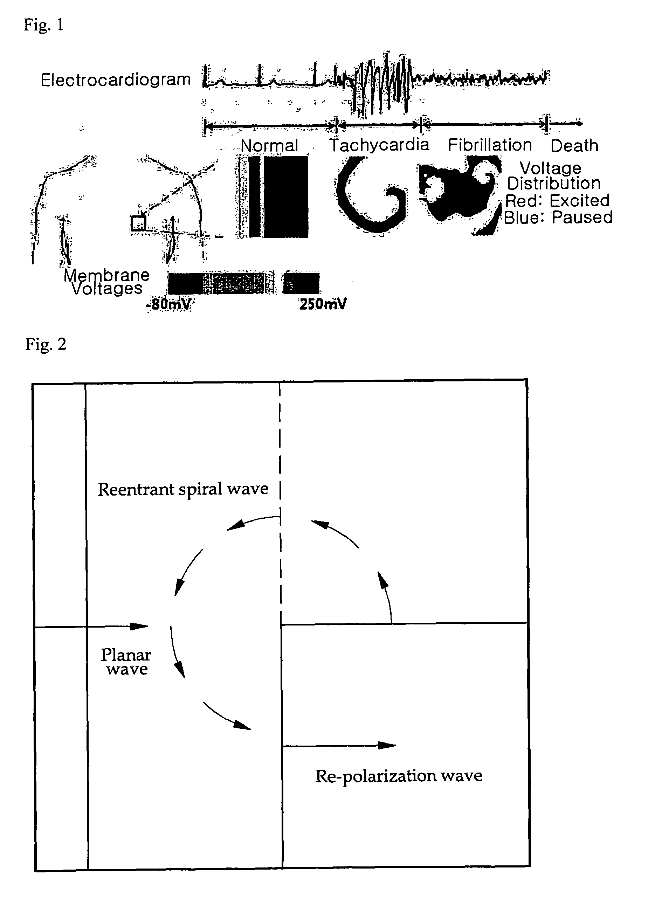

As shown in Fig. 1, the heart may suffer from bradycardia in which electric stimulation or electric conduction is irregular and slow, tachycardia in which electric stimulation or electric conduction is fast, or fibrillation in which the heart makes rapid uncoordinated twitching movement. The bradycardia, the tachycardia and the fibrillation are collectively called arrhythmia. At the present time, no arrhythmia diagnosing method is available. It is the best to merely presume that a cardiopath would probably suffer from arrhythmia.

In most cases, doctors find arrhythmia after the onset thereof and try to treat the arrhythmia. Nowadays, 0.7% of the world citizen suffers from arrhythmia. Seven hundred and twenty thousands of arrhythmia patients are generated every year. The arrhythmia treatment market is larger than eighteen billion dollars only in the U.S.A. and is growing continuously. Pursuant to statistics, about 55% of cardiopaths are died of arrhythmia after all. In order for the heart to normally eject the blood and to circulate the blood through the body, it is necessary to comply with two conditions. The first condition is that the cardiac cells have to maintain synchronism in contraction. The blood contained in the heart can be pumped out with a strong force only if all the cardiac cells are contracted synchronously. In order for the cardiac cells to be contracted synchronously, the electric excitation of the cardiac cells should occur substantially at the same time. The second condition is that the cardiac ventricles must have a sufficiently long period of pause after contraction. The amount of the blood remaining in the cardiac ventricles after contraction of the latter is quite small. If the cardiac ventricles are contracted in this state, only a small amount of the blood circulates through the body. This necessarily leads to ischemia causing shortage in the tissues.

For that reason, the cardiac ventricles should have a sufficiently long period of pause after contraction so that the blood can be sufficiently filled from the lung into the heart. To this end, there should exist a sufficiently long electric pause period after the cardiac cells are electrically excited and contracted. From this view point, the major preconditions for the heart to function normally are the synchronous contraction of the cardiac cells and the existence of the sufficiently long pause period. There are many kinds of arrhythmia. Among them, the arrhythmia attributable to an electric wave tornado generated in the cardiac tissues becomes a severe threat to the life. In the event that an electric wave tornado having a single center is generated and sustained in the heart, the cardiac cells making up the cardiac tissues are continuously electrically excited with no pause period. If the electric wave tornado is generated, the cardiac cells are constantly and continuously excited with no electric pause period. This results in rapid cardiac pulsation, which is called tachycardia. If the electric wave tornado is divided into a plurality of small tornados due to any causes, the synchronism in the electric excitation of the cardiac tissues is destroyed. This is the fibrillation occurring in a cardioplegia process. Unless the fibrillation is stopped immediately, the patient may come to death. In a nutshell, if the fibrillation occurs, the cardiac pulsation becomes fast and the pause period disappears, consequently destroying the synchronism in the electric excitation of the cardiac tissues. As a result, it is no longer possible for the heart to eject the blood, which may lead to brain death or medical death.

Despite a myriad of studies conducted thus far, no one has succeeded in providing a diagnostic basis for the electric wave tornado generated in the heart and the division of the electric wave tornado into small ones. While diagnosis and examination items for diabetes, kidney disorders, coronary artery disorders, cancers and so forth are currently available, there is no diagnosis item for diagnosing and predicting arrhythmia. In reality, a person who looks very healthy may suffer from severe arrhythmia. It is often the case that a person shows very healthy pulsation even if the heart is heavily stretched. This means that the arrhythmia such as tachycardia or fibrillation is quite irregular and is hard to predict.

Summary of the Invention

In view of the problems noted above, it is an object of the present invention to provide an arrhythmia diagnosing method and device capable of predicting and diagnosing an electric wave tornado, one of causes of arrhythmia, through the use of a non-dimensional parameter, capable of finding out patents who may possibly be died of arrhythmia or who may possibly come to brain death due to arrhythmia and capable of significantly reducing the mortality rate of patients who suffer from arrhythmia.

According to one aspect of the present invention, there is provided an arrhythmia diagnosing method, including the steps of: (a) measuring a heart dimension; (b) measuring a frequency of a cardiac electric wave; (c) measuring a conduction velocity of the cardiac electric wave; and (d) determining occurrence or absence of arrhythmia using the heart dimension, the frequency of the cardiac electric wave and the conduction velocity of the cardiac electric wave measured in steps (a), (b) and (c).

According to another aspect of the present invention, there is provided an arrhythmia diagnosing device, including: a first measuring unit for measuring a heart dimension; a second measuring unit for measuring a frequency of a cardiac electric wave; a third measuring unit for measuring a conduction velocity of the cardiac electric wave; and a determination unit for determining occurrence or absence of arrhythmia using the heart dimension, the frequency of the cardiac electric wave and the conduction velocity of the cardiac electric wave measured by the first measuring unit, the second measuring unit and the third measuring unit.

According to a further aspect of the present invention, there is provided an arrhythmia diagnosing device, including: an interface unit for making data communication with an external device; and a determination unit for determining occurrence or absence of arrhythmia based on a heart dimension, a frequency of a cardiac electric wave and a conduction velocity of the cardiac electric wave inputted through the interface unit.

According to a still further aspect of the present invention, there is provided an arrhythmia diagnosing device, including: a first measuring unit for measuring a heart dimension; a second measuring unit for measuring a heart and outputting an electrocardiogram signal; and a determination unit for calculating a frequency of a cardiac electric wave using the electrocardiogram signal, calculating a conduction velocity of the cardiac electric wave using the electrocardiogram signal or using the electrocardiogram signal and the heart dimension, and determining occurrence or absence of arrhythmia based on the heart dimension, the frequency of the cardiac electric wave and the conduction velocity of the cardiac electric wave.

According to a yet still further aspect of the present invention, there is provided an arrhythmia diagnosing device, including: an interface unit for making data communication with an external device; and a determination unit for calculating a frequency of a cardiac electric wave using an electrocardiogram signal inputted through the interface unit, calculating a conduction velocity of the cardiac electric wave using the electrocardiogram signal or using the electrocardiogram signal and a heart dimension, and determining occurrence or absence of arrhythmia based on the heart dimension, the frequency of the cardiac electric wave and the conduction velocity of the cardiac electric wave.

With the present invention, it is possible to predict and diagnose an electric wave tornado, one of causes of arrhythmia, through the use of a non-dimensional parameter, to find out patents who may possibly be died of arrhythmia or who may possibly come to brain death due to arrhythmia and to significantly reduce the mortality rate of patients who suffer from arrhythmia.

Brief Description of the Drawings

Fig. 1 is a view for explaining tachycardia and fibrillation appearing in an arrhythmia patient.

Fig. 2 is a view for explaining the influence of an S2 wave on an S1 wave in a two-dimensional imaginary heart model.

Fig. 3 is a view showing the propagation tendency of an action voltage depending on the lapse of time when the shim numbers [Sh] are 200, 400 and 600 in the two-dimensional imaginary heart model.

Fig. 4 is a view showing the propagation tendency of an action voltage depending on the lapse of time when the shim numbers [Sh] are 800, 1000 and 2000 in the two-dimensional imaginary heart model.

Fig. 5 is a view showing the propagation tendency of an action voltage depending on the lapse of time when the shim numbers [Sh] are 300, 800 and 1000 in the two-dimensional imaginary heart model having unstable cell tissues.

Fig. 6 shows images of a normal cell model and a disturbed cell model when a specified time has lapsed after application of conditions for causing tachycardia.

Fig. 7 is a schematic block diagram showing an arrhythmia diagnosing device according to one embodiment of the present invention.

Fig. 8 is a schematic block diagram showing an arrhythmia diagnosing device according to another embodiment of the present invention.

Fig. 9 is a schematic block diagram showing an arrhythmia diagnosing device according to a further embodiment of the present invention.

Detailed Description of the Preferred Embodiments

Certain preferred embodiments of an arrhythmia diagnosing method and device according to the present invention will now be described in detail.

Derivation of Non-dimensional Number

[0016]   A dominant equation describing a conduction phenomenon of an action voltage (corresponding to electric excitation) in cardiac tissues is represented by the following formula 1. The formula 1 is an equation putting the electric activities of cardiac cells together and describing an electric wave phenomenon in terms of tissues

[Formula 1]

$$\frac{\partial V}{\partial t} = -\frac{I_{ion} + I_{stim}}{C_m} + D\,\nabla^2 V$$

In the formula 1, the V is an inter-membrane voltage (mV) of myocardial cells, the $I_{ion}$ is a current per unit area (pA/cm$^2$) which is the sum of inter-membrane currents of the myocardial cells, the $I_{stim}$ is a stimulating current applied from outside, the $C_m$ is a capacitance per unit area (pF/cm$^2$) which is a capacitor component of a cell membrane, the p is a resistivity ($\Omega\cdot$cm) of a cell, the S is a surface area-to-volume ratio (cm$^{-1}$) of the cell, [x1] and the $\nabla$ is a symbol indicating a spatial derivative. The term of the left side in the formula lindicates a time-dependent change of a voltage in a specific position of a ventricular tissue. The first term of the right side in the formula 1 is a source term indicating electric excitation generated in cardiac cells making up a tissue. The second term of the right side in the formula 1 is an electric wave diffusion term. The formula 1 is a partial differential equation of reaction-diffusion type which is very typical. In addition, the D is a conductivity coefficient which is equal to 1/$\rho$SC$_m$, meaning an electric conductivity diffusion coefficient. If calculation is performed by giving substantial boundary conditions and initial conditions to the dominant equation like the formula 1, it becomes possible to quantitatively calculate an action voltage propagation phenomenon in the heart. In the formula 1, the principal dimension of the respective terms is V/t (velocity/time).

In order to analyze electric wave conduction occurring in the cardiac tissues, the equation of the formula 1 is converted to a non-dimensional equation as represented by the following formula 2. In general, it is necessary to make an equation dimensionless in order to reduce a physical phenomenon relying on a multiplicity of parameters to a question involving a small number of composite parameters. By making an equation dimensionless, it is possible to know how to simplify the physical phenomenon to a physical system. In other words, if the formula 1 as a partial differential equation is made dimensionless, the non-dimensional number appearing as coefficients of the respective terms become representative parameters defining the physical phenomenon described by the equation. Even in an equation describing cardiac electric wave conduction, it is possible to obtain a non-dimensional number by making the equation dimensionless. The non-dimensional number should become an important determination criterion in determining the generation and change of the physical phenomenon, cardiac electric wave conduction.

If a typical process of making an equation dimensionless is applied to the formula 1, it is possible to obtain a dimensionless equation as represented by the following formula 1 and to find one non-dimensional number Sh.

[Formula 2]

$$\frac{\partial V^*}{\partial t^*} = -\left( I^*_{ion} + I^*_{stim} \right) + \frac{1}{[Sh]}\nabla^2 V^*$$

In the formula 2, $V^* = V/\overline{V}$, $\nabla^* = L\overline{V}$, $t^*=t/T$ and $I^* = I/\bar{I}$, all which are non-dimensional parameters. In this regard,

the $\bar{V}$ is a characteristic voltage, the *L* is a characteristic length of the heart (a square root of a heart surface area or a cubic root of a heart volume), the *T* denotes a characteristic time which is represented by L/v, and the *v* is a conduction velocity (cm/s) of the action voltage. Accordingly, the *T* is the time required for an electric pulse to reach the size *L* of the tissues. The $\bar{l}$ denotes a characteristic current which is represented by $\bar{V}/\rho l C_m(A_s/A)$. In this regard, the *l* is a length of a unit myocardial cell, the $A_s$ is a surface area of the myocardial cell, and the *A* is a cross-sectional area of the myocardial cell.

In the formula 2, the parameters of the dimensionless dominant equation include a shim number [Sh] represented by the following formula 3.

[Formula 3]

$$[Sh] = \rho S\, C_m vL = \frac{vL}{D} = \frac{f \cdot L^2}{D} = \frac{f \cdot L}{CV}$$

In the formula 3, the f is a frequency of a cardiac electric wave, the L denotes a characteristic length of the heart, and the CV signifies a conduction velocity which is defined by *D/L.*

The physical meaning of the shim number [Sh] is as follows: *[Sh] = f·L/CV* = the inertia of an electric wave tending to be propagated in a given direction / the conduction tendency of an electric wave (the tendency of an electric wave to be uniformly dispersed with no directivity).

If the shin number grows larger, the inertia of the electric wave becomes greater, which leads to an increased probability of generation of an electric wave tornado. If the shin number grows smaller, the electric wave tends to be uniformly dispersed to the base. This reduces the directivity of the electric wave and consequently increases the stability of the electric wave.

Use of Non-dimensional Number in Diagnosing Arrhythmia

The formula 1 stated above is a dominant equation of a cardiac electric wave. In the formula 2 as a non-dimensional equation, a non-dimensional number [*Sh*] is derived by making the formula 1 dimensionless. For that reason, the cardiac electric wave is essentially decided by the non-dimensional number [*Sh*]. The cardiopathological re phenomenon [X2] depends on the non-dimensional number [*Sh*]. In other words, the non-dimensional number [*Sh*] can be used in predicting and diagnosing the risk of tachycardia and fibrillation which are severe symptoms of arrhythmia. Even if the heart is normal and does not suffer from arrhythmia, the non-dimensional number [*Sh*] may be used as a basis for predicting and determining whether the electric wave is normally generated in the cardiac tissues. More specifically, the non-dimensional number [*Sh*] would be changed in the early myocardial hypertrophy incurred by different causes such as aging and obesity. It might be possible to reversely infer the myocardial hypertrophy from the non-dimensional number [*Sh*]. The non-dimensional number [*Sh*] can also be used in quantizing the treatment effects of arrhythmia patients. By comparing the non-dimensional numbers [*Sh*] before and after treatment or medication, it becomes possible to quantitatively determine the recovery of the arrhythmia patients.

In a clinic, arrhythmia can be diagnosed by measuring the non-dimensional number [*Sh*] specific to a patient and predicting the tendency of propagation of cardiac electricity. If the heart has a pathological problem, this would affect the non-dimensional number [*Sh*]. For example, if the myocardial hypertrophy accompanied in a heart disease is pathological, there are generated a large number of connecting tissues called fibroblasts which are very low in electric conductivity. If such is the case, the possibility of generation of arrhythmia becomes very high. The conduction velocity v affects the shim number and, consequently, changes the shim number. In other words, the generation of arrhythmia is closely associated with the shim number.

The conduction velocity and the diffusion coefficient in the shim number can be measured by an electrocardiography or a catheter insertion method. The characteristic length *L* of a tissue and the characteristic length *l* of a cell can be found by reading out images taken by an imaging device such as a computed tomography device, a sonic computed tomography device or an electron microscope.

Preparatory Consideration through Computer Simulation

In the subject application, discussion will be made by two-dimensionally simplifying the heart surface tissues. While the real cardiac muscles have three-dimensional characteristics and anisotropy in the electric conductivity of tissues, the two-dimensional analysis is extensively used as a model for analyzing heart diseases. In the present embodiment, analysis was conducted with respect to a two-dimensional tissue as disclosed in Tusscher K.H., D. Noble, P.J. Noble and A.V. Panfilov, A model for Human Ventricular Tissue, Am J Physiol Heart Circ. Physiol, 2004, 286 (4), p. H1573-89. The focus of discussion made herein is to analyze whether tachycardia or fibrillation is changed depending on the shim number in the heart tissue.

As shown in Fig. 2, the imaginary heart is modeled into a rectangular shape. An S1 wave as a planar wave is propagated from the left side to the right side. An external disturbing S2 wave as a reentrant spiral wave affects the S1 wave, thereby generating a re-polarization wave.

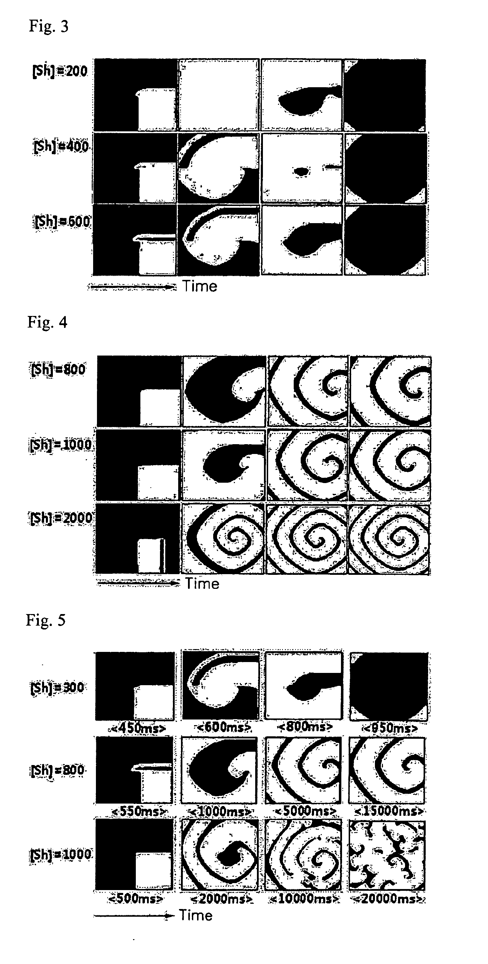

The propagation tendency of an action voltage in the imaginary heart depending on the shim number is shown Figs. 3 through 6. Referring to Fig. 3, it can be noted that, even if an external disturbing wave is applied to give tachycardia-causing conditions to the imaginary hearts having the shim numbers [*Sh*] of 200, 400 and 600, the influence of the external disturbing wave disappears after a specified time has lapsed. Referring to Fig. 4, it can be appreciated that, when an external disturbing wave is applied to give tachycardia-causing conditions to the imaginary hearts having the shim numbers [Sh] of 800, 1000 and 2000, the influence of the external disturbing wave does not appear even after the lapse of a specified time and tachycardia is generated.

Referring to Fig. 5, different results can be obtained when an external disturbing wave is applied to the imaginary hearts having the shim numbers [*Sh*] of 300, 800 and 1000 and having the instable cells added with disturbance of an ion current. More specifically, the imaginary heart having the shim number [Sh] of 300 comes back to a normal state after the lapse of 950 ms. The imaginary heart having the shim number [*Sh*] of 800 fails to come back to the normal state even after the lapse of 1500 ms, as a result of which tachycardia is generated. In the imaginary heart having the shim number [*Sh*] of 1000, tachycardia is induced with ease after the lapse of 2000 ms. It can be seen that the tachycardia is developed to fibrillation after the lapse of 20000 ms. These results indicate that the generation of arrhythmia can be predicted using the shim number [*Sh*] specific to a patient. From the simulation results stated above, it can be appreciated that the shim number [*Sh*] is a decisive factor in generating and maintaining tachycardia and fibrillation.

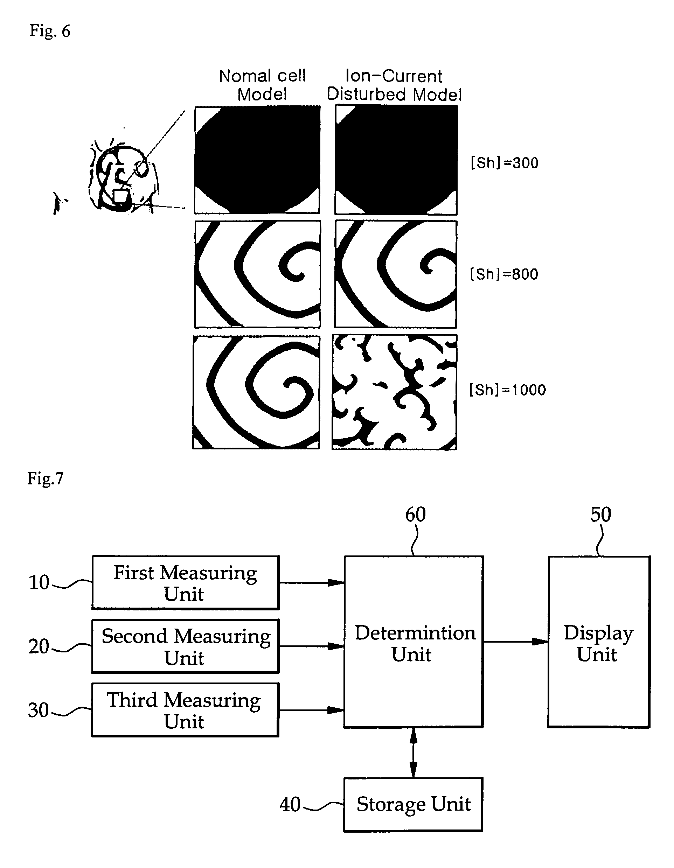

Fig. 6 shows images of a normal cell model and a disturbed cell model when a specified time has lapsed after application of conditions for causing tachycardia. Referring to Fig. 6, it can be confirmed that, even if the shim number [*Sh*] is identical, the possibility of generation of tachycardia and fibrillation differs largely depending on whether the heart model is a normal cell model or a disturbed cell model. It also can be noted that the possibility of generation of tachycardia and fibrillation becomes higher as the shim number [*Sh*] grows larger. For reference, the colors in the images shown in Figs. 3 through 6 indicate the voltage distribution in a cell membrane. The red color indicates an excited state and the blue color indicates a pause period.

As set forth above, the non-dimensional number [*Sh*] is a very important parameter in diagnosing arrhythmia. Description will now be made on a method and device for diagnosing arrhythmia through the use of the non-dimensional number [*Sh*].

Fig. 7 is a schematic block diagram showing an arrhythmia diagnosing device according to one embodiment of the present invention. [X3] Referring to Fig. 7, the arrhythmia diagnosing device according to the present embodiment includes a first measuring unit 10, a second measuring unit 20, a third measuring unit 30, a storage unit 40, a display unit 50 and a determination unit 60.

The first measuring unit 10 serves to measure a heart dimension *L*, i.e., the length of a route through which an electric wave passes in the heart. The heart dimension *L* is proportional to the size of the heart. The heart dimension *L* is also called a characteristic length of the heart.

The first measuring unit 10 is similar to or identical with an echocardiograph and is configured to measure the heart dimension *L*. The first measuring unit 10 may directly measure the heart dimension or may measure the heart volume, if necessary. In case where the heart volume is measured by the first measuring unit 10, the heart dimension *L* is calculated by the first measuring unit 10 as a cubic root of the measured heart volume as represented by the following formula 4.

[Formula 4]

$$L = l, L = \sqrt[3]{V}$$

where the *L* is a heart dimension, the *l* is a heart dimension or a heart length measured by the first measuring unit 10, and the *V* is a heart volume measured by the first measuring unit 10.

The second measuring unit 20 serves to measure a frequency *f* of a cardiac electric wave. The second measuring unit 20 is similar to or identical with an electrocardiograph for outputting an electrocardiogram signal and is configured to measure a frequency of a cardiac electric wave. The electrocardiogram signal contains R waves and a time interval between the R waves. The reciprocal number of the time interval is the frequency of the cardiac electric wave as can be seen in the following formula 5. The calculation according to the formula 5 is performed by the second measuring unit 20.

[Formula 5]

$$f = \frac{1}{RRinterval}$$

where the $RRi_{nterval}$ is a time interval between R waves contained in the electrocardiogram signal.

The third measuring unit 30 serves to measure a conduction velocity CV of a cardiac electric wave. The conduction velocity *CV* is calculated in the following manner using the electrocardiogram signal alone or the electrocardiogram signal and the heart dimension.

First, the conduction velocity *CV* of the cardiac electric wave in the ventricle is calculated by the following formula 6.

[Formula 6]

$$CV = \frac{1}{QRS(\text{or } RS)}$$

where the QRS is an interval of a QRS contained in the electrocardiogram signal, the RS is an interval of an RS contained in the electrocardiogram signal, and the *CV* is a conduction velocity of a cardiac electric wave.

Next, the conduction velocity of the cardiac electric wave in the atrium is calculated by the following formula 7.

[Formula 7]

$$CV = \frac{L}{P}$$

where the *L* is a heart dimension and the *P* is a width of a P wave contained in the electrocardiogram signal.

The third measuring unit 30 has the configurations of an echocardiograph and an electrocardiograph. The third measuring unit 30 is configured to calculate and output the conduction velocity of the cardiac electric wave using the electrocardiogram signal and the heart dimension.

A reference value for the determination of arrhythmia is stored in the storage unit 40. The reference value is used to compare the magnitude thereof with the non-dimensional number [*Sh*]. The reference value may be set to have many different values. In the present embodiment, the reference value is set to have a specific value in a range of 800 to 1200. The reference value can be changed by a user depending on a variety of factors such the age, sex, health, dietary habit, weight and stature of a patient.

The display unit 50 serves to display the operating state of the arrhythmia diagnosing device. The display unit 50 is formed of an LCD and is capable of displaying the non-dimensional number [*Sh*] and the occurrence or absence of arrhythmia. The display unit 50 may be formed of a green LED and a red LED so that the red LED can be turned on in case of arrhythmia being diagnosed and the green LED can be turned on in case of arrhythmia not being diagnosed.

The determination unit 60 determines the occurrence of absence of arrhythmia pursuant to the heart dimension, the frequency of the cardiac electric wave and the conduction velocity of the cardiac electric wave inputted from the first measuring unit 10, the second measuring unit 20 and the third measuring unit 30. The determination unit 60 outputs a control signal corresponding to the determination result to the display unit 50.

First, the determination unit 60 calculates the non-dimensional number [*Sh*] according to the following formula 8.

[Formula 8]

$$[Sh] = \frac{f \cdot L}{CV}$$

where the [$Sh$] is a non-dimensional number, the $f$ is a frequency of a cardiac electric wave, the $L$ is a heart dimension, and the $CV$ is a conduction velocity of a cardiac electric wave.

Thereafter, the determination unit 60 compares the calculated non-dimensional number [$Sh$] with the reference value stored in the storage unit 40. If the calculated non-dimensional number [$Sh$] is equal to or larger than the reference value, the determination unit 60 determines that the heart suffers from arrhythmia. If the calculated non-dimensional number [$Sh$] is smaller than the reference value, the determination unit 60 determines that the heart does not suffer from arrhythmia. The determination unit 60 outputs a control signal corresponding to the determination result to the display unit 50. Responsive to the control signal, the display unit 50 displays the occurrence or absence of arrhythmia.

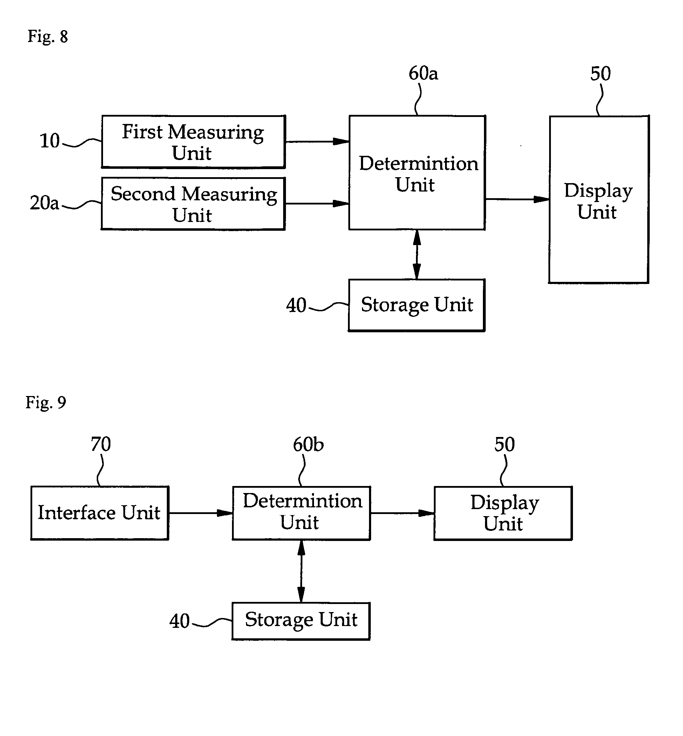

Fig. 8 is a schematic block diagram showing an arrhythmia diagnosing device according to another embodiment of the present invention. Referring to Fig. 8, the arrhythmia diagnosing device according to the present invention includes a second measuring unit 20a formed of an electrocardiograph. Accordingly, the second measuring unit 20a outputs an electrocardiogram signal. As in the foregoing embodiment, the first measuring unit 10 serves to measure a heart dimension. Needless to say, it may be possible to measure a heart volume instead of the heart dimension. In this case, the heart dimension is calculated by the first measuring unit 10 or the determination unit 60a.

The determination unit 60a calculates a frequency of a cardiac electric wave and a conduction velocity thereof using the heart dimension and the electrocardiogram signal inputted. Based on this calculation result, the determination unit 60a calculates the non-dimensional number [Sh]. By comparing the calculated non-dimensional number [Sh] with the reference value, the determination unit 60a determines the occurrence or absence of arrhythmia.

Fig. 9 is a schematic block diagram showing an arrhythmia diagnosing device according to a further embodiment of the present invention. Referring to Fig. 9, the arrhythmia diagnosing device according to the present embodiment includes an interface unit 70, a storage unit 40, a display unit 50 and a determination unit 60b.

The interface unit 70 is provided to make wire communication or wireless communication with an echocardiograph or an electrocardiograph. The heart dimension measured by the echocardiograph and the electrocardiogram signal (containing an RRinterval, a QRS interval, an RS interval and a P wave width) outputted from the electrocardiograph are inputted to the determination unit 60b through the interface unit 70.

As in the foregoing embodiment, the determination unit 60b calculates a non-dimensional number [$Sh$] using the values inputted through the interface unit 70. By comparing the calculated non-dimensional number [$Sh$] with the reference value, the determination unit 60a determines the occurrence or absence of arrhythmia. The determination unit 60b outputs a control signal so that the determination result can be displayed on the display unit 50. If necessary, the arrhythmia diagnosing device may be configured such that the heart dimension, the frequency of the cardiac electric wave and the conduction velocity of the cardiac electric wave are inputted through the interface unit 70.

While certain preferred embodiment of the invention has been described hereinabove, the present invention is not limited thereto. It is to be understood that various changes and modifications may be made without departing from the scope of the invention defined in the claims.

**Claims**

1. An arrhythmia diagnosing method, comprising the steps of:

   (a) measuring a heart dimension;
   (b) measuring a frequency of a cardiac electric wave;
   (c) measuring a conduction velocity of the cardiac electric wave; and
   (d) determining occurrence or absence of arrhythmia using the heart dimension, the frequency of the cardiac electric wave and the conduction velocity of the cardiac electric wave measured in steps (a), (b) and (c).

2. The method of claim 1, wherein, in step (d), a non-dimensional number is obtained by processing the heart dimension, the frequency of the cardiac electric wave and the conduction velocity of the cardiac electric wave measured in steps (a), (b) and (c) and the non-dimensional number is used in determining the occurrence or absence of arrhythmia.

**3.** The method of claim 2, wherein the non-dimensional number is calculated by:

<Formula 8>

$$[Sh] = \frac{f \cdot L}{CV},$$

where the [Sh] is the non-dimensional number, the *f* is the frequency of the cardiac electric wave, the *L* is the heart dimension, and the *CV* is the conduction velocity of the cardiac electric wave.

**4.** The method of claim 3, wherein it is determined in step (d) that a heart suffers from arrhythmia, if the non-dimensional number [Sh] is equal to or larger than a predetermined reference value.

**5.** The method of any one of claims 2 to 4, wherein the frequency of the cardiac electric wave is measured using an electrocardiogram signal generated by an electrocardiograph and the conduction velocity of the cardiac electric wave is measured using the electrocardiogram signal generated by the electrocardiograph or using the electrocardiogram signal and the heart dimension.

**6.** The method of claim 5, wherein the heart dimension is calculated by

<Formula 4>

$$L = l, L = \sqrt[3]{V},$$

the frequency of the cardiac electric wave is calculated by

[Formula 5]

$$f = \frac{1}{RRinterval},$$

and
the conduction velocity of the cardiac electric wave is calculated by

[Formula 6]

$$CV = \frac{1}{QRS(\text{or } RS)}$$

or

[Formula 7]

$$CV = \frac{L}{P}$$

,

in the formulas 4 through 7, the 1 being a heart dimension measured by an echocardiograph, the *V* being a heart volume measured by the echocardiograph, the $RR_{interval}$ being a time interval between R waves contained in the electrocardiogram signal, the QRS being an interval of a QRS contained in the electrocardiogram signal, the RS being an interval of an RS contained in the electrocardiogram signal, and the *P* being a width of a P wave contained in the electrocardiogram signal,
the formula 6 being applied to a ventricle and the formula 7 being applied to an atrium.

7. The method of claim 3, wherein the heart dimension is calculated by

<Formula 4>

$$L = l, L = \sqrt[3]{V}$$

,

the frequency of the cardiac electric wave is calculated by

[Formula 5]

$$f = \frac{1}{RRinterval}$$

,

and
the conduction velocity of the cardiac electric wave is calculated by

[Formula 6]

$$CV = \frac{1}{QRS(\text{or } RS)}$$

or

[Formula 7]

$$CV = \frac{L}{P}$$

,

in the formulas 4 through 7, the 1 being a heart dimension measured by an echocardiograph, the V being a heart volume measured by the echocardiograph, the $RR_{interval}$ being a time interval between R waves contained in the electrocardiogram signal, the QRS being an interval of a QRS contained in the electrocardiogram signal, the RS being an interval of an RS contained in the electrocardiogram signal, and the *P* being a width of a P wave contained in the electrocardiogram signal,

the formula 6 being applied to a ventricle and the formula 7 being applied to an atrium,

determination being made that a heart suffers from arrhythmia, if the non-dimensional number [Sh] is larger than 800 to 1200.

8.  An arrhythmia diagnosing device, comprising:

a first measuring unit for measuring a heart dimension;
a second measuring unit for measuring a frequency of a cardiac electric wave;
a third measuring unit for measuring a conduction velocity of the cardiac electric wave; and
a determination unit for determining occurrence or absence of arrhythmia using the heart dimension, the frequency of the cardiac electric wave and the conduction velocity of the cardiac electric wave measured by the first measuring unit, the second measuring unit and the third measuring unit.

9.  An arrhythmia diagnosing device, comprising:

an interface unit for making data communication with an external device; and
a determination unit for determining occurrence or absence of arrhythmia based on a heart dimension, a frequency of a cardiac electric wave and a conduction velocity of the cardiac electric wave inputted through the interface unit.

10. The device of claim 8 or 9, wherein the determination unit calculates a non-dimensional number by processing the heart dimension, the frequency of the cardiac electric wave and the conduction velocity of the cardiac electric wave and determines the occurrence or absence of arrhythmia based on the non-dimensional number thus calculated.

11. The device of claim 10, wherein the non-dimensional number is calculated by:

<Formula 8>

$$[Sh] = \frac{f \cdot L}{CV},$$

where the [*Sh*] is the non-dimensional number, the *f* is the frequency of the cardiac electric wave, the *L* is the heart dimension, and the *CV* is the conduction velocity of the cardiac electric wave.

12. The device of claim 11, wherein the determination unit determines that a heart suffers from arrhythmia, if the non-dimensional number [Sh] is equal to or larger than a predetermined reference value.

13. The device of claim 11, wherein the frequency of the cardiac electric wave is measured using an electrocardiogram signal and the conduction velocity of the cardiac electric wave is measured using the electrocardiogram signal or using the electrocardiogram signal and the heart dimension.

14. The device of claim 13, wherein the heart dimension is calculated by

<Formula 4>

$$L = l, L = \sqrt[3]{V}$$

the frequency of the cardiac electric wave is calculated by

[Formula 5]

$$f = \frac{1}{RRinterval}$$

and
the conduction velocity of the cardiac electric wave is calculated by

[Formula 6]

$$CV = \frac{1}{QRS(\text{or } RS)}$$

or

[Formula 7]

$$CV = \frac{L}{P}$$

in the formulas 4 through 7, the *l* being a heart dimension measured by an echocardiograph, the *V* being a heart volume measured by the echocardiograph, the $RR_{interval}$ being a time interval between R waves contained in the electrocardiogram signal, the QRS being an interval of a QRS contained in the electrocardiogram signal, the RS being an interval of an RS contained in the electrocardiogram signal, and the P being a width of a P wave contained in the electrocardiogram signal, the formula 6 being applied to a ventricle and the formula 7 being applied to an atrium.

15. The device of claim 14, wherein the determination unit determines that a heart suffers from arrhythmia, if the non-dimensional number [Sh] is larger than 800 to 1200.

16. An arrhythmia diagnosing device, comprising:

a first measuring unit for measuring a heart dimension;
a second measuring unit for measuring a heart and outputting an electrocardiogram signal; and
a determination unit for calculating a frequency of a cardiac electric wave using the electrocardiogram signal, calculating a conduction velocity of the cardiac electric wave using the electrocardiogram signal or using the electrocardiogram signal and the heart dimension, and determining occurrence or absence of arrhythmia based on the heart dimension, the frequency of the cardiac electric wave and the conduction velocity of the cardiac electric wave.

**17.** An arrhythmia diagnosing device, comprising:

an interface unit for making data communication with an external device; and
a determination unit for calculating a frequency of a cardiac electric wave using an electrocardiogram signal inputted through the interface unit, calculating a conduction velocity of the cardiac electric wave using the electrocardiogram signal or using the electrocardiogram signal and a heart dimension, and determining occurrence or absence of arrhythmia based on the heart dimension, the frequency of the cardiac electric wave and the conduction velocity of the cardiac electric wave.

**18.** The device of claim 16 or 17, wherein the determination unit calculates a non-dimensional number by processing the heart dimension, the frequency of the cardiac electric wave and the conduction velocity of the cardiac electric wave and determines the occurrence or absence of arrhythmia based on the non-dimensional number thus calculated.

**19.** The device of claim 18, wherein the non-dimensional number is calculated by:

<Formula 8>

$$[Sh] = \frac{f \cdot L}{CV},$$

where the [Sh] is the non-dimensional number, the f is the frequency of the cardiac electric wave, the L is the heart dimension, and the CV is the conduction velocity of the cardiac electric wave.

**20.** The device of claim 19, wherein the determination unit determines that a heart suffers from arrhythmia, if the non-dimensional number [Sh] is equal to or larger than a predetermined reference value.

**21.** The device of claim 19, wherein the frequency of the cardiac electric wave is calculated by

[Formula 5]

$$f = \frac{1}{RRinterval},$$

and
the conduction velocity of the cardiac electric wave is calculated by

[Formula 6]

$$CV = \frac{1}{QRS(or\ RS)}$$

or

[Formula 7]

$$CV = \frac{L}{P},$$

in the formulas 5 through 7, the $RR_{interval}$ being a time interval between R waves contained in the electrocardiogram signal, the QRS being an interval of a QRS contained in the electrocardiogram signal, the RS being an interval of an RS contained in the electrocardiogram signal, and the P being a width of a P wave contained in the electrocardiogram signal,

the formula 6 being applied to a ventricle and the formula 7 being applied to an atrium.

22. The device of claim 21, wherein the determination unit determines that a heart suffers from arrhythmia, if the non-dimensional number [Sh] is larger than 800 to 1200.

Fig. 1

Electrocardiogram

Normal   Tachycardia   Fibrillation   Death

Voltage
Distribution
Red: Excited
Blue: Paused

Membrane
Voltages

-80mV                    250mV

Fig. 2

Reentrant spiral wave

Planar
wave

Re-polarization wave

Fig. 3

[Sh]=200

[Sh]=400

[Sh]=600

Time

Fig. 4

[Sh]=800

[Sh]=1000

[Sh]=2000

Time

Fig. 5

[Sh]=300

<450ms> <600ms> <800ms> <950ms>

[Sh]=800

<550ms> <1000ms> <5000ms> <15000ms>

[Sh]=1000

<500ms> <2000ms> <10000ms> <20000ms>

Time

Fig. 6

Fig.7

Fig. 8

Fig. 9

**REFERENCES CITED IN THE DESCRIPTION**

**Non-patent literature cited in the description**

- **TUSSCHER K.H. ; D. NOBLE ; P.J. NOBLE ; A.V. PANFILOV.** A model for Human Ventricular Tissue. *Am J Physiol Heart Circ. Physiol,* 2004, vol. 286 (4), H1573-89